# EUROPEAN PATENT SPECIFICATION

(11) **EP 3 774 855 B1**
(45) Date of publication and mention of the grant of the patent: **28.08.2024**
(21) Application number: 19728908.5
(22) Date of filing: 28.05.2019
(51) Int. Cl.: C07K 14/31

(54) **AN ANTIMICROBIAL COMPOSITION FOR SELECTIVELY INHIBITING GROWTH OF P. ACNES BACTERIA**
EINE ANTIMIKROBIELLE ZUSAMMENSETZUNG ZUR SELEKTIVEN WACHSTUMSHEMMUNG VON P. ACNES BAKTERIEN
COMPOSITION ANTIMICROBIENNE INHIBANT LA CROISSANCE DE P. ACNES

(30) Priority: 13.06.2018 EP 18177459
(43) Date of publication of application: 17.02.2021
(73) Proprietor: Unilever Global IP Limited, Wirral, Merseyside CH62 4ZD (GB); Unilever IP Holdings B.V., 3013 AL Rotterdam (NL)
(72) Inventor: MITRA, Rupak, Bangalore 560 066 (IN); MUKHERJEE, Sayandip, Bangalore 560 066 (IN); PATHAK, Sandip Bhanudas, Bangalore 560 066 (IN); SARKAR, Samarpita, Burdwan 713 304 West Bengal (IN)
(74) Representative: James, Helen Sarah
(86) International application number: PCT/EP2019/063765
(87) International publication number: WO 2019/238409

(56) References cited:
- US-B1- 7 572 602
- TAMMY L BANNERMAN ET AL: "Staphylococcus capitis subsp. ureolyticus subsp. nov. from Human Skin", INTERNATIONAL JOURNAL OF SYSTEMATIC BACTERIOLOGY, 1 January 1991 (1991-01-01), England, pages 144 - 147, XP055493126, Retrieved from the Internet <URL:http://www.microbiologyresearch.org/docserver/fulltext/ijsem/41/1/ijs-41-1-144.pdf?expires=1531827570&id=id&accname=guest&checksum=FB35627BED7F96AC0B6914829EA191E5> DOI: 10.1099/00207713-41-1-144
- EWA JONCZYK-MATYSIAK ET AL: "Prospects of Phage Application in the Treatment of Acne Caused by Propionibacterium acnes", FRONTIERS IN MICROBIOLOGY, vol. 8, 8 February 2017 (2017-02-08), XP055494102, DOI: 10.3389/fmicb.2017.00164
- "Advances in Virus Research", vol. 83, 1 January 2012, ACADEMIC PRESS, US, ISSN: 0065-3527, article DANIEL C. NELSON ET AL: "Endolysins as Antimicrobials", pages: 299 - 365, XP055220045, DOI: 10.1016/B978-0-12-394438-2.00007-4

## Description

### Field of the invention

The present invention relates to a method and a composition to prevent or treat acne by selective inhibition of *P. acnes* bacteria. The method comprises treating skin with an isolate of another bacteria present as a minor contributor of the normal skin flora.

### Background of the invention

Acne, also known as Acne vulgaris, is a common skin condition that affects nearly all adolescents and adults at some point in their lives. It has a complex etiology, involving abnormal keratinization, excess sebum production, androgen function, bacterial growth, and immune hypersensitivity. Although one or more of the above processes is correlated with acne, the one triggering factor and the exact sequence of events leading to the formation of acne lesions has not been fully understood. Other factors which have been linked to acne are presence of free radicals with subsequent oxidative stress leading to cellular damage. It has been observed that acne usually occurs in areas rich in sebaceous glands like the face, neck and back.

A bacteria *Propionibacterium acnes (P. acnes)* has also been implicated in occurrence of acne. It is one of the important and dominant bacteria residing on the human facial skin surface. *P. acnes* is an aerotolerant anaerobe, slow-growing, rod shaped Gram-positive bacteria. It resides in the sebaceous glands and it constitutes an important part of the skin commensal microbiota. *P. acnes* uses sebum and by-product from surrounding skin tissue as sources of energy and nutrients. This results in some fatty acid release which can irritate the follicle wall and induce inflammation, leading to acne or acne vulgaris. Acne vulgaris is a chronic, inflammatory disorder of the pilosebaceous gland. It affects almost all adolescents at some point of their lives with 15-20% suffering from moderate to severe forms of acne.

Acne has been treated in many ways. Most treatments take several weeks to months before a noticeable change is seen. Benzoyl peroxide which has an antibacterial effect has been used for mild cases of comedones. In very severe cases of acne, antibiotics like tetracycline, erythromycin and clindamycin have been used. Antibiotics are believed to work by several mechanisms, the most important being the decrease in the number of bacteria in and around the follicle. They are also thought to reduce the irritating chemicals produced by the white blood cells in the sebum, thereby reducing the inflammatory response. However, the drawback of antibiotics and other sort of general antimicrobial treatment is that they are broad-spectrum and help in killing or inhibiting most of the bacteria on skin.

Most of the treatments, as summarized above, involve use of synthetic chemicals. Many people do not prefer use of synthetic chemicals since they are believed to be harsh on the human body. Some people believe that synthetic chemicals cause side effects. Hence, more and more people prefer use of materials which are "natural" e.g. actives based on herbal origin. Further, these days, the actives based on microbiological or biotechnological origin is also coming to be considered as more "natural" then purely synthetic approaches used so far.

Skin, human body's largest organ is colonized by diverse microorganisms. Most of the microorganisms are considered either beneficial or opportunistic potential pathogens . Recent advances in DNA sequencing and computational biology are helping us to identify and understand the beneficial role of these microorganisms on our skin to a greater resolution.

Interactions between skin microorganisms and the human host can be classified into three clusters: 1. Commensalism (one benefits, and no harm occurs to the other), 2. Mutualism (both find benefit), 3. Parasitism (one organisms benefit and other is harmed). Microbes which are not often associated with disease, are mainly known as commensals. Increasing evidence shows that commensal organisms on skin help the immune system to fight against pathogens and maintain homeostasis of the microbiome. One example is *Staphylococcus epidermidis* (a Gram-positive coagulase negative staphylococci) which plays an important role in acne inhibition. *Staphylococcus epidermidis* mediates fermentation of glycerol which inhibit overgrowth of *P. acnes,* leading to acne inhibition. Thus the approach to use broad spectrum antibiotic action which kills or inhibits all the microorganisms on the skin is finding lesser and lesser acceptance among health care practitioners. Increasingly, the approach to selective kill or inhibit microorganism on skin, is finding favour among health care practitioners. Further, the use of antibiotics in treatment of antibiotic resistant strains of *P. acnes,* is ineffective.

Thus there is a need for finding novel methods of treating problems such as acne in selective fashion. The present inventors thus started working towards providing "natural" solutions to solving the problem of acne. They took up the approach of testing new actives that kill or selectively inhibit growth of *P. acnes* to the exclusion of other organisms like *S. epidermidis, E. coli and S. aureus* and experimented with the same. They finally hit up on an isolate (extracellular factor) of *Staphylococcus capitis ureolyticus (SCU),* a minor commensal contributor of the skin flora, that was found to selectively inhibit the growth of *P. acnes.*

US2016346294 (Vyome Biosciences) titled 'Treatments for resistant acne' relates generally to novel molecules, compositions, and formulations for treatment of bacterial infections in general and more specifically to bacterial infections with antibiotic resistant pathogens. Exemplified therein is S. *capitis* (subspecies not specifically mentioned) which is considered a pathogen, not an antimicrobial.

Bannerman and Kloos (International journal of systematic bacteriology, 1991, p 144-147; XP55493126) discloses that a new subspecies, *Staphylococcus capitis* subsp. *ureolyticus,* was isolated from human skin and is described on the basis of studies of 15 to 26 strains. DNA-DNA reassociation reactions demonstrated that these strains were closely related to *Staphylococcus capitis* but were significantly divergent. The strains of *S*. *capitis* subsp. *ureolyticus* can be distinguished from S. *capitis* by their positive urease activity, their ability to produce acid from maltose under aerobic conditions, their fatty acid profile, and their colony morphology. The type strain of the new subspecies is strain ATCC 49326.

Jończyk-Matysiak et al (Frontiers in Microbiology, 2017, 8, 164; XP55494102) Propionibacterium acnes is associated with purulent skin infections, and it poses a global problem for both patients and doctors. Acne vulgaris (acne) remains a problem due to its chronic character and difficulty of treatment, as well as its large impact on patients' quality of life. Due to the chronic course of the disease, treatment is long lasting, and often ineffective. Currently there are data regarding isolation of P. acnes phages, and there have been numerous studies on phage killing of P. acnes, but no data are available on phage application specifically in acne treatment. In this review, we have summarized the current knowledge on the phages active against P. acnes described so far and their potential application in the treatment of acne associated with P. acnes. The treatment of acne with phages may be important in order to reduce the overuse of antibiotics, which are currently the main acne treatment. However, more detailed studies are first needed to understand phage functioning in the skin microbiome and the possibility to use phages to combat P. acnes.

It is thus an object of the present invention to provide for selective kill or inhibition of *P. Acnes* to treat acne.

It is another object of the present invention to provide solution to treating acnes through more natural or nature based actives.

### Summary of the invention

According to the first aspect of the present invention there is provided an antimicrobial composition for selectively inhibiting growth of *P. Acnes* bacteria comprising
(i) Isolate of *Staphylococcus capitis ureolyticus* (SCU); and
(ii) a topically acceptable carrier;

wherein the Isolate of *Staphylococcus capitis ureolyticus* is characterized with one or more of urease activity, maltose fermentation test, or 16s rDNA sequencing; and
wherein the topically acceptable carrier comprises an anhydrous base, a gel, lotion, cream or emulsion and
wherein the composition additionally comprises *P. acnes* phage-derived endolysins and nucleic acid molecules encoding the same; and
wherein the isolate of SCU is prepared using a process comprising the steps of:
   (i) Shaking SCU in a liquid broth at a temperature of 35 to 37°C;
   (ii) Separating the supernatant free of the cells;
   (iii) Concentrating the supernatant by precipitating with ammonium sulphate;
   (iv) Resuspending the precipitate of step (iii) with a phosphate buffer and glycerol
to prepare the desired isolate of SCU; and
wherein the nucleotide sequence of endolysin is SEQ ID1:

ATGCGTTATATTCCGGCAGCACATCATTCAGCAGGTAGCAATCATCCGGTTAATCGTGTTGTTATTCATGCAACCTGTCCGG
ATGTTGGTTTTCCGAGCGCAAGCCGTAAAGGTCGTGCAGTTAGCACCGCAAACTATTTTGCAAGCCCGAGCAGCGGTGGT
AGCGCACATTATGTTTGTGATATTGGTGAAACCGTTCAGTGTCTGAGCGAAGGCACCATTGGTTGGCATGCACCGCCTAAT
CCGCATAGCCTGGGTATTGAAATTTGTGCAGATGGTGGTAGCCATGCAAGCTTTCGTGTTCCGGGTCATGCATATACCCGT
GAACAGTGGCTGGATCCGCGTGTTTGGCCTGCAGTTGAAAAAGCAGCAATTCTGTGTCGTCGTCTGTGCGATAAATACAAT
GTTCCGAAACGTAAACTGAGCGCAGCAGATCTGAAAGCAGGTCGTCGTGGTGTTTGTGGTCATGTTGATGTTACCGATGCA
TGGCATCAGAGCGATCATGATGATCCGGGTCCGTGGTTTCCGTGGGATCGTTTTATGGCAGTTGTTAATGGTCATAACGAA
AGCGGTGAACTGACCGTTGCAGATGTTAAAGCACTGCATGATCAGATTAAACAGCTGAGCGCACAGCTGGCAGGTAGCGT
TAATAAACTGCATCATGATGTTGGTGTTGTTCAGGTTCAGAATGGTGATCTGGGTAAACGTGTTGATGCACTGAGCTGGGT
TAAAAATCCGGTTACCGGTAAACTGTGGCGTACCAAAGATGCACTGTGGTCAGTTTGGTATTATGTTCTGGAATGTCGTAG
CCGTATTGATCGTCTGGAAAGCGCAGTTAATGGTCTGAAAAAACTCGAGCACCACCACCACCACCACTGAGATCCGGCTG
CTAA.

According to a second aspect, the present invention relates to a composition of the first aspect for use in controlling or eradicating *P. Acnes* from skin.

According to a third aspect, the present invention relates to a composition of the first aspect for use in reducing or eliminating acne on skin.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

The composition as per this invention could be in the form of a leave-on or wash-off format meant for cleaning or disinfecting topical areas e.g. skin and/or hair of mammals, especially humans. Such a composition includes any product applied to a human body for also improving appearance, cleansing, odor control or general aesthetics. The composition of the present invention may be in the form of a liquid, lotion, cream, foam or gel, or toner, or applied with an implement or via a face mask, pad or patch. "Skin" as used herein is meant to include skin on the face and body (e.g., neck, chest, back, arms, underarms, hands, legs, buttocks and scalp). It is especially useful for treatment of acne on the face or any other part of the body where acne forms.

The present invention relates to an antimicrobial composition for selectively inhibiting growth of *P. Acnes* bacteria comprising (i) Isolate of *Staphylococcus capitis ureolyticus* (SCU); and(ii) a topically acceptable carrier.

*Staphylococcus capitis* is a minor contributor of the normal flora that resides on the human face. *Staphylococcus capitis* is a Gram-positive coagulase negative staphylococcus. In the present invention the inventors found that *S. capitis ureolyticus* a subspecies of *S. capitis* secretes a proteinaceous factor which inhibits the growth of *P*. *acne,* and *P. granulosum* but not *S. epidermidis, E. coli and S*. *aureus.* Thus, it is envisaged that this selective/targeted reduction of *Propionibacterium* could be an effective prebiotic/probiotic approach to control *P. acnes* population within acne vulgaris while maintaining the balance of the facial skin microbiome.

The Bacterial strain and growth condition used in the present invention were as follows: *Staphylococcus capitis* clinical isolate strain (SCU, isolated from the face of a volunteer). The strains were revived using tryptic soya broth and tryptic soya agar under aerobic condition at 37°C.

The clinical isolate of Staphylococcus capitis was confirmed by 16s rDNA sequencing.

Primers used for sequencing are
A1: 5' AGAGTTTGATCCTGGCTCAG
A5: 5' GGGTTGCGCTCGTTG
A9: 5' ATCCAAGCTTAAGGAGGTGA

Clinical isolate SCU was identified as *S*. *capitis ureolyticus* using the following procedure. Biochemical tests were done to characterize the strains of S. *capitis.* SCU showed positive for both biochemical test (urease activity and maltose fermentation test).

Urease test defines urease activity of organism which can cleave the urea, present in the medium and forming ammonia and carbon dioxide. Later, ammonia combines with carbon dioxide and water, forming ammonium carbonate which turns the medium alkaline. Because of the alkaline pH, the medium turns into pink from yellow (original colour).

Maltose fermentation test defines carbohydrate (sugar) maltose fermentation by microbe. Maltose fermentation led acid production as by product which turns the medium acidic. Because of acidic pH medium turns to yellow from red (original colour). According to the Kloos classification, *Staphylococcus capitis subsp. ureolyticus* can be distinguished from other subspecies of S. *capitis* by their positive urease activity and maltose fermentation.

Thus, SCU prepared for this invention is a clinical isolate of *Staphylococcus capitis subspecies ureolyticus.*

The isolate of SCU as per this invention is preferably prepared using a process comprising the steps of (i) shaking SCU in a liquid broth at a temperature of 35 to 37°C; (ii) separating the supernatant free of the cells; (iii) concentrating the supernatant by precipitating with ammonium sulphate; and (iv) resuspending the precipitate of step (iii) with a phosphate buffer and glycerol to prepare the desired isolate of SCU.

The preferred process is as given below. Supernatant of SCU was prepared by shaking SCU in a liquid broth at 35 to 37 °C overnight. The cell free supernatant was prepared by centrifuging this overnight culture at about 10000g for about 10-20 mins, preferably about 15 minutes. To the cell free supernatant about 75% ammonium sulfate was added under constant stirring overnight at about 4°C. The next day ammonium sulphate treated supernatant was centrifuged at about 10000g for about 30 mins and the pellet obtained was re-suspended in 1X PBS (pH 7.0) with about 10 % glycerol. Overnight dialysis was carried out to remove the ammonium sulphate against PBS buffer and 10% glycerol. The next day the dialyzed fractions were aliquoted and stored in -80°C till further use.

By Isolate of *Staphylococcus capitis ureolyticus* (SCU) as per this invention is meant the supernatant of a culture of SCU. Thus, the term "isolate of *Staphylococcus capitis ureolyticus* (SCU)" and the term "supernatant of a SCU culture" may be used interchangeably throughout this specification and claims. One process to prepare such a supernatant of a SCU culture has been described in the two preceding paragraphs. The process hereinabove described makes it amply clear as to the meaning of the term "Isolate of *Staphylococcus capitis ureolyticus* (SCU)". Isolate of SCU as per this invention is not meant SCU that has been merely isolated from skin as is disclosed in Tammy L Bannerman et al, "Staphylococcus capitis subsp. ureolyticus subsp. nov. from Human Skin", International Journal of Systematic Bacteriology, 1 January 1991, pages 144-147, XP055493126.

The composition as per the present invention may additionally comprise *P. acnes* phage-derived endolysins and nucleic acid molecules encoding the same.

In addition to the isolate of SCU that provides the selective antimicrobial efficacy as per the first aspect of the present invention, the present inventors were looking to seek improvement over this technology. They looked at various other approaches and arrived at the application of bacteriophages and bacteriophage-derived enzymes towards this end. A group of phage-derived enzymes are peptidoglycan (PG) hydrolases known as endolysins. Endolysins are novel muralytic hydrolases encoded by double stranded DNA phages which degrade the PG layer of the bacterial cell wall thereby allowing the progeny phages to escape in the late phase of the infection cycle. Purified endolysins when exposed to PG externally can cause "lysis from outside". Based on their antimicrobial properties (extraordinary substrate specificity and high activity when added externally) endolysins from phages infecting Gram-positive pathogens has been the motivation and hypothesis for the present inventors to improve the effect exhibited by the first aspect of the present invention.

The present inventors, without wishing to be bound by theory, believe that the application of *P. acnes* phage-derived endolysins offer a promising route towards this since antimicrobial resistance to endolysin has not been recorded. They have successfully expressed and purified a recombinant form of *P. acnes* phage endolysin using an *E. coli* based heterologous expression system and have demonstrated its ability to selectively lyse *P. acnes* in-vitro. The endolysin does not target any of the regular bacterial flora found on human skin namely *S. aureus, S. epidermidis or E. coli.*

Thus, it is useful that the endolysin that is included is a recombinant form of *P. acnes* phage endolysin. This endolysis is preferably cloned from endolysin gene sequence (Gene ID: NC_018851; 855 nucleotide base pair long, 284 amino acids, protein ID: 97935.1) from *Propionibacterium* phage 29399B_C (GenBank: JX262225.1) which is codon optimized for expression in *E*. *coli* and cloned into commercially available pET303/CT-His expression vector.

The nucleotide sequence of endolysin which is especially useful is as per the sequence ID SEQ ID1 which is listed below:

ATGCGTTATATTCCGGCAGCACATCATTCAGCAGGTAGCAATCATCCGGTTAATCGTGTTG
TTATTCATGCAACCTGTCCGGATGTTGGTTTTCCGAGCGCAAGCCGTAAAGGTCGTGCAGT
TAGCACCGCAAACTATTTTGCAAGCCCGAGCAGCGGTGGTAGCGCACATTATGTTTGTGAT
ATTGGTGAAACCGTTCAGTGTCTGAGCGAAGGCACCATTGGTTGGCATGCACCGCCTAAT
CCGCATAGCCTGGGTATTGAAATTTGTGCAGATGGTGGTAGCCATGCAAGCTTTCGTGTTC
CGGGTCATGCATATACCCGTGAACAGTGGCTGGATCCGCGTGTTTGGCCTGCAGTTGAAA
AAGCAGCAATTCTGTGTCGTCGTCTGTGCGATAAATACAATGTTCCGAAACGTAAACTGAG
CGCAGCAGATCTGAAAGCAGGTCGTCGTGGTGTTTGTGGTCATGTTGATGTTACCGATGCA
TGGCATCAGAGCGATCATGATGATCCGGGTCCGTGGTTTCCGTGGGATCGTTTTATGGCA
GTTGTTAATGGTCATAACGAAAGCGGTGAACTGACCGTTGCAGATGTTAAAGCACTGCATG
ATCAGATTAAACAGCTGAGCGCACAGCTGGCAGGTAGCGTTAATAAACTGCATCATGATGT
TGGTGTTGTTCAGGTTCAGAATGGTGATCTGGGTAAACGTGTTGATGCACTGAGCTGGGTT
AAAAATCCGGTTACCGGTAAACTGTGGCGTACCAAAGATGCACTGTGGTCAGTTTGGTATT
ATGTTCTGGAATGTCGTAGCCGTATTGATCGTCTGGAAAGCGCAGTTAATGGTCTGAAAAA
ACTCGAGCACCACCACCACCACCACTGAGATCCGGCTGCTAA.

An especially preferred aspect relates to the endolysin where the stop codon was removed from the 3' end of the gene to accommodate a 6X Histidine tag.

Polypeptide sequence of Endolysin with C-terminal Histidine tag (x6)

MRYIPAAHHSAGSNHPVNRVVIHATCPDVGFPSASRKGRAVSTANYFASPSSGGSAHYVCDIG
ETVQCLSEGTIGWHAPPNPHSLGIEICADGGSHASFRVPGHAYTREQWLDPRVWPAVEKAAIL
CRRLCDKYNVPKRKLSAADLKAGRRGVCGHVDVTDAWHQSDHDDPGPWFPWDRFMAVVNG
HNESGELTVADVKALHDQIKQLSAQLAGSVNKLHHDVGVVQVQNGDLGKRVDALSWVKNPVT
GKLWRTKDALWSVWYYVLECRSRIDRLESAVNGLKKLEHHHHHH

It is also possible that the nucleic acid molecules of the endolysin for optional inclusion in the composition of the invention comprise fragments, variants and fusions of the endolysin which are capable of specifically binding to and/or lysing cells of *P. acnes.*

The composition of the invention includes a topically acceptable carrier which may comprise an anhydrous base, a gel, a lotion, a cream or an emulsion.

The composition of the invention is preferably a wash-off composition and this is enabled by including 1 to 80% by weight of a surfactant. In general, the surfactants may be chosen from the surfactants described in well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used.

A particularly preferred surfactant is soap. Soap is a suitable surfactant for personal washing applications of composition of the invention. The soap is preferably C8-C24 soap, more preferably C10-C20 soap and most preferably C12-C16 soap. The soap may or may not have one or more carbon-carbon double bond or triple bond. The cation of the soap may be alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc.

A typical fatty acid blend consisted of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions. The most preferred soap is a laurate soap. The soap, when present in solid forms of the present invention is present in an amount of 30 to 90%, preferably from 50 to 85%, more preferably 55 to 75% by weight of the composition. The soap, when present in liquid forms of the composition is present in 0.5 to 20%, preferably from 1 to 10% by weight of the composition.

Alternately the surfactants are non-ionic surfactants, such as C8-C22, preferably C8-C16 fatty alcohol ethoxylates, comprising between 1 and 8 ethylene oxide the surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16. Suitable surfactant concentrations in liquid forms of cleaning application are generally more than 0.5 but less than 10%, preferably from 1 to 5 % by weight of the composition. In solid compositions, the surfactant is preferably present in 5 to 40%, preferably from 10 to 30% by weight of the composition.

Water may preferably be present in 10 to 90% by weight of the composition depending on the format of the composition. In solid composition water may be present in 10-30%, while in liquid or semi-solid composition, water may be present in 40 to 90%.

The composition of the invention is especially suitable for use in a wash off process where the contact time of the antimicrobial actives with the surface is low, i.e of the order of less than 5 minutes, preferably less than 2 minutes, further more preferably less than a minute and in many cases less than 15 seconds.

When the composition in accordance with the invention is a leave on composition, it preferably comprises one or more of fragrance, surfactant, organic sunscreen, inorganic sunscreen, emollient, humectant, extender pigment and preservative.

Sunscreens include those materials which block harmful ultraviolet light. Preferred suncreens are the derivatives of p-aminobenzoic acid (PABA), cinnamate and salicylate. For example, avobenzophenone (Parsol^{®} 1789), octyl methoxycinnamate and 2-hydroxy-4-methoxy benzophenone (also known as oxybenzone) can be used. Octyl methoxycinnamate and 2-hydroxy-4-methoxy benzophenone are commercially available under the trade marks, Parsol^{®} MCX and Benzophenone-3, respectively. Ecamsule^{®}, a benzylidene camphor derivative, and drometrizole trisiloxane, a benzotriazole, may also be used. Further examples include Octocrylene, phenylbenzimidazole sulfonic acid (also known as Ensulizole^{®}), ethylhexyl salicylate, diethylhexyl naphthylate, bimotrizinole (trade marked as Tinosorb^{®} S) and bisoctrizole (Tinosorb^{®} M). Inorganic sunscreens include oxides like titanium dioxide and zinc oxide which reflect or scatter the sunrays. The quantity of sunscreens present in the compositions could vary depending upon the degree of protection desired from UV radiation. Preferably, the compositions comprise 0.01 to 15 % by weight, more preferably 0.1 to 10 and most preferably 0.5 to 7.5 % by weight sunscreen.

Illustrative examples of the types of fragrances that may be used include those comprising terpenes and terpene derivatives like those described in Bauer, K., et al., Common Fragrance and Flavor Materials, VCH Publishers (1990). Further examples include myrcene, dihydromyrenol, citral, tagetone, cis-geranic acid, citronellic acid, mixtures thereof.

The carrier acts as diluent or dispersant for the ingredients of the compositions. The carrier may be aqueous-based, anhydrous or an emulsion, whereby a water-in-oil or oil-in-water emulsion is generally preferred. If the use of water is desired, water typically makes up the balance of the composition, which most preferably is from 40 to 80 % by weight of the composition.

In addition to water, organic solvents may optionally be included as carrier to assist any other carrier in the compositions of the present invention. Examples include alkanols like ethyl and isopropyl alcohol.

Other suitable organic solvents include ester oils like isopropyl myristate, cetyl myristate, 2-octyldodecyl myristate, avocado oil, almond oil, olive oil and neopentylglycol dicaprate. Typically, such ester oils assist in emulsifying the compositions, and an effective amount is often used to yield a stable, and most preferably, water-in-oil emulsion.

Emollients may also be used, if desired, as a carrier. Alcohols like 1 -hexadecanol (i.e. cetyl alcohol) are preferred. Other emollients include silicone oils and synthetic esters. Silicone oils suitable for use include cyclic or linear polydimethylsiloxanes containing from 3 to 9, preferably from 4 to 5 silicon atoms. Non-volatile silicone oils useful as emollients include polyalkyl siloxanes, polyalkylaryl siloxanes and polyether siloxane copolymers. The non-volatile polyalkyl siloxanes useful polydimethylsiloxanes. Silicone elastomers may also be used. The ester emollients that may optionally be used are:
(i) alkenyl or alkyl esters of fatty acids having 10 to 20 carbon atoms. Examples thereof include isoarachidyl neopentanoate, isononyl isonanonoate, oleyl myristate, oleyl stearate, and oleyl oleate;
(ii) ether-esters such as fatty acid esters of ethoxylated fatty alcohols;
(iii) polyhydric alcohol esters. Ethylene glycol mono and di-fatty acid esters, diethylene glycol mono- and di-fatty acid esters, polyethylene glycol (200- 6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters;
(iv) wax esters such as beeswax, spermaceti, stearyl stearate and arachidyl behenate; and,
(v) sterols esters, of which cholesterol fatty acid esters are examples.

Emollients, when present, typically make up from 0.1 to 50 % by weight of the composition, including all ranges subsumed therein.

Fatty acids having from 10 to 30 carbon atoms may also be included as carriers. Examples of such fatty acids include pelargonic, lauric, myristic, palmitic, stearic, isostearic, oleic, linoleic, arachidic, behenic or erucic acid and mixtures thereof.

Humectants of the polyhydric alcohol type may also be employed in the compositions. The humectant often aids in increasing the effectiveness of the emollient, reduces scaling at the skin surface, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results, the humectant is preferably propylene glycol or sodium hyaluronate. Other humectants which may be used include hydroxyethyl urea. The amount of humectant may be 0.2 to 25 % by weight and preferably from 0.5 to 15 % by weight of the composition.

Moisturisation may be improved through use of petrolatum or paraffin. Thickeners may also be utilized as a portion of the carrier in the compositions. Typical thickeners include cross-linked acrylates (e.g. Carbopol^{®} 982), hydrophobically-modified acrylates (e.g. Carbopol^{®}1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.001 to 5, optimally from 0.01 to 0.5 % by weight of the composition.

Surfactants may also be present. When present, the total amount of surfactants is 2 to 40 % by weight, and preferably from 4 to 20 % by weight, optimally from 5 to 12 % by weight of the composition. The surfactant is selected from the group consisting of anionic, nonionic, cationic and amphoteric actives. Particularly preferred nonionic surfactants are those with a C₁₀₋₂₀ fatty alcohol or acid hydrophobe condensed with from 2 to 100 moles of ethylene oxide or propylene oxide per mole of hydrophobe; mono- and di- fatty acid esters of ethylene glycol; fatty acid monoglyceride; sorbitan, mono- and di-C8-C20 fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C_{8 to 20} acyl isethionates, acyl glutamates, C_{8 to 20} alkyl ether phosphates and combinations thereof.

Various other ingredients may also be used in compositions. Actives are defined as skin benefit agents other than emollients and other than ingredients that merely improve the physical characteristics of the composition. Although not limited to this category, general examples include extender pigments such as talcs and silicas, as well as alpha- hydroxy acids, beta-hydroxy acids and zinc salts.

Beta-hydroxy acids include salicylic acid. Zinc oxide and zinc pyrithione are examples of useful zinc salts.

Compositions, especially those containing water, need to be protected against harmful microorganisms. Anti-microbial compounds, such as triclosan, and preservatives may become necessary. Suitable preservatives include alkyl esters of p-hydroxybenzoic acid, hydantoin derivatives, propionate salts, and a variety of quaternary ammonium compounds. Particularly preferred preservatives are methyl paraben, propyl paraben, phenoxyethanol and benzyl alcohol. Preservatives are from 0.1 to 2 % by weight of the composition.

The packaging could be a patch, bottle, tube, roll-ball applicator, propellant driven aerosol device, squeeze container or lidded jar.

According to another aspect of the present invention there is provided a method of controlling or eradicating *P. Acnes* from skin comprising the step of applying a composition of the first aspect on to a desired skin surface. The method is preferably non-therapeutic. The method is especially useful for reducing or eliminating acne on skin.

The invention will now be illustrated with the help of the following non-limiting examples.

### Examples

### Effect of the SCU isolate of the invention on inhibition of P. Acnes as compared to S. Aureus and S. epidermidis

### Preparation of P. acnes lawn

*Propionibacterium acnes ATCC* 6919 and ATCC 29399 were grown on CY agar for 3 days at 37°C under anaerobic condition. A loop of culture was re-suspended in PBS (pH 7.0) and optical density was set at 0.8 (10⁸cells/ml). 100µl of 10⁸cells/ml was spread plated onto 10 cm petri plate containing 20 ml of 1% TSA.

### Preparation of S. epidermidis , and S. aureus bacterial lawn:

*Staphylococcus aureus* 6538 and *Staphylococcus* . *epidermidis* 12228 , were grown overnight in tryptic soya agar plate. Next morning, a loop full of culture was taken in saline (0.85% NaCl) and OD was set at 0.8 (10⁸cells/ml).100µl of the above cell suspension was spread plated onto 10 cm petri plate containing 20 ml of 1% TSA.

### Spotting of Staphylococcus capitis (SCU) cell pellet

*Staphylococcus capitis ureolyticus* (SCU) was grown overnight in 10 ml TSB. Next morning, culture was centrifuged, washed with saline and re-suspended in 1ml fresh TSB. 10µl of the re-suspended culture were spotted onto plates containing *P. Acnes, S. aureus and S*. *epidermidis* lawn. Plates were incubated at 37°C for at 37°C under anaerobic condition. The effect of the SCU on *P. acnes, S. aureus and S*. *epidermidis,* is summarized in the table below as zone of inhibition (ZOI):

### Co-incubation study of Propionibacterium acnes and Ammonium sulphate treated cell free supernatant of SCU

*Propionibacterium acnes* ATCC 6919 was grown under anaerobic condition for 3 days and then OD was made to 0.8 (10⁸ cells/ml). Serial dilution was done using the above culture up to 10⁴ cell/ml. SCU ammonium sulphate treated cell free culture supernatant was used for this experiment. 100µl of diluted 10⁶ P. acnes ATCC 6919 and 100µl of ammonium sulphate treated SCU cell free culture supernatant were co-incubated in 1.5ml centrifuge tube at 37°C for 15mins. After 15mins incubation, 800µl of fresh TSB was added to the cell mixture to stop/slow down the reaction (dilution effect) immediately followed by serial dilution and plating onto 1% TSA plate and incubated anaerobically for 3 days at 37°C. A control which involving only similar P. acnes dilution with 100µl PBS buffer treatment was simultaneously set up.

The effect of the SCU on *P. Acnes,* is summarized in the table below in terms of log reduction

| *P. acnes* | *P. acnes* | *S*. *aureus* | *S*. *epidermidis* |
|---|---|---|---|
| Zone of Inhibition (ZOI) (cm) | Log reduction | Zone of Inhibition (ZOI) (cm) | Zone of Inhibition (ZOI) (cm) |
| 2.1 ± 0.15 | 4.5 | No zone of inhibition | No zone of inhibition |

The data in the table above indicates that the SCU isolate of the invention is very specific in its antimicrobial activity on *P. acnes* and is ineffective against *S*. *aureus* or *S*. *epidermidis.*

### Synergistic effect of combination of Propionibacterium phage-derived recombinant endolysin & Staphylococcus capitis ureolyticus (SCU) derived protein isolate:

*P. acnes* 6919 strain (ATCC, USA) was grown under anaerobic conditions on RCM agar from Glycerol stocks. A loopful of culture from glycerol stock was streaked on freshly prepared RCM agar plate and incubated at 37°C anaerobically for 4-5 days. After the incubation, the culture was taken from the plate and re-suspended in saline and then centrifuged at 8000 rpm for 6 minutes at 4°C. The pellet obtained was re-suspended in 10mM HEPES buffer (pH 7.0). An OD₆₀₀ culture of 0.5 was made in HEPES buffer. 1:10 dilution of 0.5 OD adjusted culture was used for contact kill assay corresponding to 10⁶ cells per ml.

The assay was done in 200µl total volume. 14 µl of purified endolysin (final conc. 100µg/ml) and 50 µl of *Staphylococcus capitis ureolyticus* #1 (SCU) derived protein (final conc. 750 µg/ml) was used in the assay. For control, 136 µl of 1:10 diluted 0.6 OD₆₀₀ adjusted culture was mixed with 64 µl of HEPES buffer to make a volume of 200 µl. Similarly, For Endolysin and SCU protein, required amounts of protein were mixed with *P. acnes* culture for individual & for combination treatment. The mixture was mixed and incubated at 37°C for 15 min in shaker incubator. Post incubation, the samples were neutralised by serial 1:10 dilution in D/E broth followed by saline dilutions. 100 µl of each dilution was plated on fresh RCM agar plates and allowed to dry. The plates were sealed with parafilm and incubated at 37°C in anaerobic chamber for 4-5 days. Post incubation, colonies were counted and titer was estimated as log CFU/ml. The relative log reductions of individual and combination treatment were calculated with respect to control and the data is presented in the table below:

| Sample | Log CFU/ml | Relative log reduction |
|---|---|---|
| Control | 7.53 | - |
| Endolysin (100 µg/ml) | 7.24 | 0.29 |
| SCU (750 µg/ml) | 4.95 | 2.58 |
| Endolysin + SCU | 3.98 | 3.55 |

The data in the table above shows synergistic antimicrobial activity of endolysin and SCU protein against *P. acnes* for a combined exposure of 15 minutes.

## Claims

1. An antimicrobial composition for selectively inhibiting growth of *P*. *acnes* bacteria comprising
(i) Isolate of *Staphylococcus capitis ureolyticus* (SCU); and
(ii) A topically acceptable carrier;
wherein the Isolate of *Staphylococcus capitis ureolyticus* is characterized with one or more of urease activity, maltose fermentation test, or 16s rDNA sequencing; and
wherein the topically acceptable carrier comprises an anhydrous base, a gel, lotion, cream or emulsion and
wherein the composition additionally comprises *P*. *acnes* phage-derived endolysins and nucleic acid molecules encoding the same; and
wherein the isolate of SCU is prepared using a process comprising the steps of:
(i) Shaking SCU in a liquid broth at a temperature of 35 to 37°C;
(ii) Separating the supernatant free of the cells;
(iii) Concentrating the supernatant by precipitating with ammonium sulphate;
(iv) Resuspending the precipitate of step (iii) with a phosphate buffer and glycerol to prepare the desired isolate of SCU; and
wherein the nucleotide sequence of endolysin is SEQ ID1:

ATGCGTTATATTCCGGCAGCACATCATTCAGCAGGTAGCAATCATCCGGTTAATCGTGTTGTTATTCATGCAACC
TGTCCGGATGTTGGTTTTCCGAGCGCAAGCCGTAAAGGTCGTGCAGTTAGCACCGCAAACTATTTTGCAAGCCC
GAGCAGCGGTGGTAGCGCACATTATGTTTGTGATATTGGTGAAACCGTTCAGTGTCTGAGCGAAGGCACCATTG
GTTGGCATGCACCGCCTAATCCGCATAGCCTGGGTATTGAAATTTGTGCAGATGGTGGTAGCCATGCAAGCTTT
CGTGTTCCGGGTCATGCATATACCCGTGAACAGTGGCTGGATCCGCGTGTTTGGCCTGCAGTTGAAAAAGCAGC
AATTCTGTGTCGTCGTCTGTGCGATAAATACAATGTTCCGAAACGTAAACTGAGCGCAGCAGATCTGAAAGCAGG
TCGTCGTGGTGTTTGTGGTCATGTTGATGTTACCGATGCATGGCATCAGAGCGATCATGATGATCCGGGTCCGT
GGTTTCCGTGGGATCGTTTTATGGCAGTTGTTAATGGTCATAACGAAAGCGGTGAACTGACCGTTGCAGATGTTA
AAGCACTGCATGATCAGATTAAACAGCTGAGCGCACAGCTGGCAGGTAGCGTTAATAAACTGCATCATGATGTT
GGTGTTGTTCAGGTTCAGAATGGTGATCTGGGTAAACGTGTTGATGCACTGAGCTGGGTTAAAAATCCGGTTAC
CGGTAAACTGTGGCGTACCAAAGATGCACTGTGGTCAGTTTGGTATTATGTTCTGGAATGTCGTAGCCGTATTGA
TCGTCTGGAAAGCGCAGTTAATGGTCTGAAAAAACTCGAGCACCACCACCACCACCACTGAGATCCGGCTGCTA
A.

2. A composition as claimed in claim 1 wherein said endolysin is recombinant form of *P*. *acnes* phage endolysin.

3. A composition as claimed in claim 1 or 2 wherein said endolysin is cloned from endolysin gene sequence Gene ID: NC_018851; 855 nucleotide base pair long, 284 amino acids, protein ID: 97935.1 from *Propionibacterium* phage 29399B_C GenBank: JX262225.1 which is codon optimized for expression in *E. coli* and cloned into commercially available pET303/CT-His expression vector.

4. A composition as claimed in claim 3 wherein the stop codon was removed from the 3' end of the gene to accommodate a 6X Histidine tag.

5. A composition as claimed in any one of the preceding claims wherein the nucleic acid molecules comprise fragments, variants and fusions of the endolysin which are capable of specifically binding to and/or lysing cells of *P*. *Acnes.*

6. A composition as claimed in any one of the preceding claims for use in controlling or eradicating *P. Acnes* from skin.

7. A composition as claimed in any one of the preceding claims 1 to 5 for use in reducing or eliminating acne on skin.

## Patentansprüche

1. Antimikrobielle Zusammensetzung zur selektiven Wachstumshemmung von *P*. *acnes Bakterien,* umfassend
(i) Isolat von *Staphylococcus capitis ureolyticus* (SCU); und
(ii) einen topisch akzeptablen Träger,
wobei das Isolat von *Staphylococcus capitis ureolyticus* durch eine oder mehrere von Urease-Aktivität, einem Maltose-Fermentierungstest oder 16s-rDNA-Sequenzierung charakterisiert ist; und
wobei der topisch akzeptable Träger eine wasserfreie Base, ein Gel, eine Lotion, eine Creme oder Emulsion umfasst und
wobei die Zusammensetzung zusätzlich *P. acnes*-Phagen-abgeleitete Endolysine und Nucleinsäuremoleküle, die diese codieren, umfasst; und
wobei das Isolat der SCU unter Verwendung eines Verfahrens hergestellt wird, umfassend die Schritte:
(i) Schütteln von SCU in einer flüssigen Brühe bei einer Temperatur von 35 bis 37°C;
(ii) Abtrennen des von Zellen befreiten Überstands;
(ii) Konzentrieren des Überstands durch Fällen mit Ammoniumsulfat;
(iv) Resuspendieren des Niederschlags von Schritt (iii) mit einem Phosphatpuffer und Glycerin, um das gewünschte Isolat von SCU herzustellen; und
wobei die Nucleotidsequenz von Endolysin die SEQ ID1 ist:

ATGCGTTATATTCCGGCAGCACATCATTCAGCAGGTAGCAATCATCCGGTTAATCGTGTTGTTATTCATGCAACC
TGTCCGGATGTTGGTTTTCCGAGCGCAAGCCGTAAAGGTCGTGCAGTTAGCACCGCAAACTATTTTGCAAGCCC
GAGCAGCGGTGGTAGCGCACATTATGTTTGTGATATTGGTGAAACCGTTCAGTGTCTGAGCGAAGGCACCATTG
GTTGGCATGCACCGCCTAATCCGCATAGCCTGGGTATTGAAATTTGTGCAGATGGTGGTAGCCATGCAAGCTTT
CGTGTTCCGGGTCATGCATATACCCGTGAACAGTGGCTGGATCCGCGTGTTTGGCCTGCAGTTGAAAAAGCAGC
AATTCTGTGTCGTCGTCTGTGCGATAAATACAATGTTCCGAAACGTAAACTGAGCGCAGCAGATCTGAAAGCAGG
TCGTCGTGGTGTTTGTGGTCATGTTGATGTTACCGATGCATGGCATCAGAGCGATCATGATGATCCGGGTCCGT
GGTTTCCGTGGGATCGTTTTATGGCAGTTGTTAATGGTCATAACGAAAGCGGTGAACTGACCGTTGCAGATGTTA
AAGCACTGCATGATCAGATTAAACAGCTGAGCGCACAGCTGGCAGGTAGCGTTAATAAACTGCATCATGATGTT
GGTGTTGTTCAGGTTCAGAATGGTGATCTGGGTAAACGTGTTGATGCACTGAGCTGGGTTAAAAATCCGGTTAC
CGGTAAACTGTGGCGTACCAAAGATGCACTGTGGTCAGTTTGGTATTATGTTCTGGAATGTCGTAGCCGTATTGA
TCGTCTGGAAAGCGCAGTTAATGGTCTGAAAAAACTCGAGCACCACCACCACCACCACTGAGATCCGGCTGCTA
A.

2. Zusammensetzung, wie im Anspruch 1 beansprucht, wobei das Endolysin eine rekombinante Form des *P. acnes*-Phagen-Endolysins ist.

3. Zusammensetzung, wie im Anspruch 1 oder 2 beansprucht, wobei das Endolysin aus der Endolysin-Gensequenz Gene ID: NC_018851; 855 Nucleotidbasenpaar lang, 284 Aminosäuren, Protein ID: 97935.1 aus der *Propionibacterium-Phage* 29399B_C GenBank: JX262225.1 geklont wurde, die für die Expression in *E. coli* Codon-optimiert ist und in den im Handel erhältlichen Expressionsvektor pEI303/CT-His geklont wurde.

4. Zusammensetzung, wie im Anspruch 3 beansprucht, wobei das Stoppcodon von dem 3'-Ende des Gens zur Aufnahme eines 6X Histidin-Tags entfernt wurde.

5. Zusammensetzung, wie in irgendeinem der vorhergehenden Ansprüche beansprucht, wobei die Nucleinsäuremoleküle Fragmente, Varianten und Fusionen des Endolysins umfassen, die in der Lage sind, *P. Acnes-Zellen* spezifisch zu binden und/oder zu lysieren.

6. Zusammensetzung, wie in irgendeinem der vorhergehenden Ansprüche beansprucht, zur Verwendung bei der Kontrolle auf oder zur Beseitigung von *P. Acnes* von der Haut.

7. Zusammensetzung, wie in irgendeinem der vorhergehenden Ansprüche 1 bis 5 beansprucht, zur Verwendung beim Reduzieren und Beseitigen von Akne auf der Haut.

## Revendications

1. Composition antimicrobienne pour inhiber sélectivement la croissance des bactéries *P. acnes,* comprenant
(i) un isolat de *Staphylococcus capitis ureolyticus* (SCU) ; et
(ii) un véhicule topiquement acceptable ;
dans laquelle l'isolat de *Staphylococcus capitis ureolyticus* est **caractérisé par** un élément ou plusieurs parmi une activité d'uréase, un test de fermentation du maltose, ou un séquençage d'ADNr 16s ; et
dans laquelle le véhicule topiquement acceptable comprend une base anhydre, un gel, une lotion, une crème ou une émulsion et
dans laquelle la composition comprend additionnellement des endolysines dérivées de phage de *P. acnes* et des molécules d'acide nucléique codant pour celles-ci ; et
dans laquelle l'isolat de SCU est préparé en utilisant un procédé comprenant les étapes de :
(i) agitation du SCU dans un bouillon liquide à une température de 35 à 37°C ;
(ii) séparation du surnageant exempt de cellules ;
(iii) concentration du surnageant par précipitation avec du sulfate d'ammonium ;
(iv) resuspension du précipité de l'étape (iii) avec un tampon de phosphate et du glycérol pour préparer l'isolat désiré de SCU ; et
dans laquelle la séquence nucléotidique de l'endolysine est la SEQ ID 1 :

ATGCGTTATATTCCGGCAGCACATCATTCAGCAGGTAGCAATCATCCGGTTAATCGTGTTGTTATTCATGCAACC
TGTCCGGATGTTGGTTTTCCGAGCGCAAGCCGTAAAGGTCGTGCAGTTAGCACCGCAAACTATTTTGCAAGCCC
GAGCAGCGGTGGTAGCGCACATTATGTTTGTGATATTGGTGAAACCGTTCAGTGTCTGAGCGAAGGCACCATTG
GTTGGCATGCACCGCCTAATCCGCATAGCCTGGGTATTGAAATTTGTGCAGATGGTGGTAGCCATGCAAGCTTT
CGTGTTCCGGGTCATGCATATACCCGTGAACAGTGGCTGGATCCGCGTGTTTGGCCTGCAGTTGAAAAAGCAGC
AATTCTGTGTCGTCGTCTGTGCGATAAATACAATGTTCCGAAACGTAAACTGAGCGCAGCAGATCTGAAAGCAGG
TCGTCGTGGTGTTTGTGGTCATGTTGATGTTACCGATGCATGGCATCAGAGCGATCATGATGATCCGGGTCCGT
GGTTTCCGTGGGATCGTTTTATGGCAGTTGTTAATGGTCATAACGAAAGCGGTGAACTGACCGTTGCAGATGTTA
AAGCACTGCATGATCAGATTAAACAGCTGAGCGCACAGCTGGCAGGTAGCGTTAATAAACTGCATCATGATGTT
GGTGTTGTTCAGGTTCAGAATGGTGATCTGGGTAAACGTGTTGATGCACTGAGCTGGGTTAAAAATCCGGTTAC
CGGTAAACTGTGGCGTACCAAAGATGCACTGTGGTCAGTTTGGTATTATGTTCTGGAATGTCGTAGCCGTATTGA
TCGTCTGGAAAGCGCAGTTAATGGTCTGAAAAAACTCGAGCACCACCACCACCACCACTGAGATCCGGCTGCTA
A.

2. Composition selon la revendication 1, dans laquelle ladite endolysine est une forme recombinante de l'endolysine de phage de *P. acnes.*

3. Composition selon la revendication 1 ou 2, dans laquelle ladite endolysine est clonée à partir de la séquence du gène d'endolysine ID : NC_018851 ; longue de 855 paires de base nucléotidique, 284 acides aminés, protéine ID : 97935.1 provenant du phage *Propionibacterium* 29399B_C GenBank : JX26225.1 qui est optimisé au niveau du codon pour l'expression dans *E. coli* et cloné dans le vecteur d'expression pET303/CT-His disponible dans le commerce.

4. Composition selon la revendication 3, dans laquelle le codon d'arrêt a été retiré de l'extrémité 3' du gène pour accueillir un marqueur 6X histidine.

5. Composition selon l'une quelconque des revendications précédentes, dans laquelle les molécules d'acide nucléique comprennent des fragments, variants et fusions de l'endolysine qui sont aptes à lier spécifiquement et/ou lyser des cellules de *P. Acnes.*

6. Composition selon l'une quelconque des revendications précédentes pour une utilisation dans la régulation ou l'éradication de *P. Acnés de* la peau.

7. Composition selon l'une quelconque des revendications précédentes 1 à 5 pour une utilisation dans la réduction ou l'élimination de l'acné sur la peau.
